(19) Europäisches Patentamt
European Patent Office
Office européen des brevets

(11) Publication number: 0 113 864
A1

(12) EUROPEAN PATENT APPLICATION

(21) Application number: 83112400.3

(22) Date of filing: 09.12.83

(51) Int. Cl.³: C 07 C 175/00, A 61 K 31/19, A 61 K 31/16, A 61 K 31/215

(30) Priority: 17.12.82 US 450624

(43) Date of publication of application: 25.07.84 Bulletin 84/30

(84) Designated Contracting States: AT BE CH DE FR GB IT LI NL SE

(71) Applicant: SCHERING CORPORATION, 2000 Galloping Hill Road, Kenilworth, New Jersey 07033 (US)

(72) Inventor: Wright, John Jessen, 691 Marigold Court, Evansville Indiana 47712 (US)

(74) Representative: Antony, Fritz, Dr. et al, P.O. Box 601 Winkelriedstrasse 35, CH-6002 Lucerne (CH)

(54) **Cyclopropyl substituted polyenes, processes for their preparation and pharmaceutical compositions containing them.**

(57) Cyclopropyl substituted polyenes of the formula

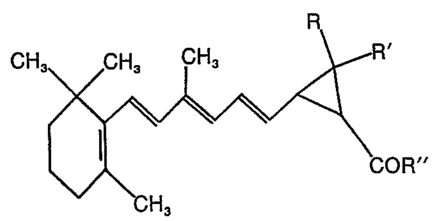

I

wherein

R and R′ are independently hydrogen atoms or lower alkyl groups;

R″ is a hydroxy or lower alkoxy group or a group –NHR‴ wherein R‴ is a hydrogen atom or a lower alkyl group;

and the pharmaceutically acceptable salts, formed with cations, of those compounds of formula I wherein R″ is a hydroxy group;

wherein "lower alkyl" and "lower alkoxy" mean straight and branched chain alkyl and alkoxy groups that contain from 1 to 6 carbon atoms, are useful in the treatment of dermatoses. Compositions containing them and processes for their preparation are also disclosed.

EP 0 113 864 A1

CYCLOPROPYL SUBSTITUTED POLYENES, PROCESSES FOR THEIR PREPARATION AND PHARMACEUTICAL COMPOSITIONS CONTAINING THEM

This invention relates to cyclopropyl substituted polyenes, to methods for their preparation and to pharmaceutical compositions containing them as the active agent. These polyenes are novel compounds useful for treating dermatoses in warm-blooded animals.

The invention provides compounds of the formula

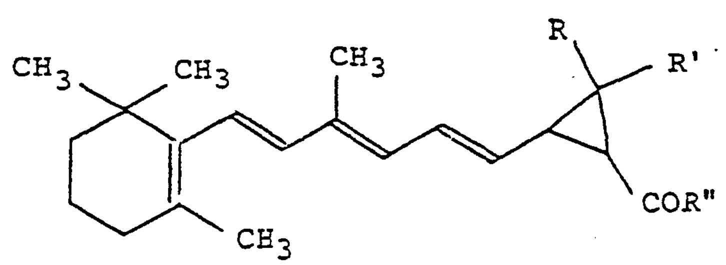

I

wherein

R and R' are independently hydrogen atoms or lower alkyl groups;

R" is a hydroxy or lower alkoxy group or a group -NHR''' wherein R''' is a hydrogen atom or a lower alkyl group;

and the pharmaceutically acceptable salts, formed with cations, of those compounds of formula I wherein R" is a hydroxy group;

wherein "lower alkyl" and "lower alkoxy" mean straight and branched chain alkyl and alkoxy groups that contain from 1 to 6 carbon atoms.

The lower alkyl and lower alkoxy groups may for example be methyl, ethyl, propyl, butyl, tertiary butyl and isopropyl groups and the corresponding alkoxy groups. The cations in the pharmaceutically acceptable salts must themselves be pharmaceutically acceptable, e.g. an alkali metal such as sodium or potassium, or ammonium salts such as diethanolamine or N-methylglucamine salts.

Particularly preferred compounds of this invention include: 4-E-[2,3-trans-isopropylidene-7-methyl-9-(2,6,6-trimethylcyclohexen-1-yl)]nona-4,6,8-trienoic acid; and 4-Z-[2,3-trans-isopropylidene-7-methyl-9-(2,6,6-trimethylcyclohexen-1-yl)]nona-4,6,8-trienoic acid.

The compounds of formula I are useful in the topical and systemic treatment of dermatoses and systemic treatment of inflammation, for example acne and psoriasis and other keratinisation disorders that are accompanied by an increased or pathologically altered cornification in warm-blooded animals, including humans. The compounds of this invention can be used for topical and systemic therapy of benign and malignant neoplasia or premalignant lesions or epithelial cancers as well as for the systemic and topical prophylaxis of these conditions.

The anti-acne activity of the compounds of this invention was measured in an animal model for measuring comedolytic activity. In this model, retinoic acid, an effective anti-acne agent, is known to be active; and the compounds of this invention were found to have activity and potency similar to those of retinoic acid. For example, when 4-E-[2,3-trans-isopropylidene-7-methyl-9-(2,6,6-trimethylcyclohexen-1-yl)]nona-4,6,8-trienoic acid was applied topically it was effective at a daily dose of 0.02 mg., as was retinoic acid.

In this test, the test compound was applied topically to a circumscribed area of skin on the posterior dorsal aspect of the mouse. Twenty-four hours following the last

application, portions of the skin from treated and untreated areas were excised, fixed in formalin and processed for histological assessment.

Psoriasis is characterised by increased epidermipoiesis associated with a high mitotic rate, rapid cell turnover and altered keratinisation. The psoriatic epidermis can be normalised by slowing down cell growth through inhibiting mitosis.

The anti-psoriatic activity of the compounds of this invention was measured in an animal model for measuring the effect of the test compounds on mitotic rate in croton-oil-stimulated epidermis. In this model the compounds of this invention were found to be active. For example, when 4-E-[2,3-trans-isopropylidene-7-methyl-9-(2,6,6-trimethyl-cyclohexen-1-yl)]nona-4,6,8-trienoic acid was applied topically it was effective at a daily dose of 0.1 mg.

In this test, a procedure modified from Belman et al., Cancer Research 32, 450-454 (1972), the test compound was applied topically to a circumscribed area of skin on the posterior dorsal aspect of the mouse with a croton oil solution. Controls were treated only with croton oil. Twenty-four hours later the rats were injected (i.p.) with colcemid in saline to block cells entering metaphase. Four hours later skin from the treated areas was excised and processed for histology. The results indicated that the test compounds effectively reduced mitosis.

The compounds of this invention can be used as medicaments in the form of pharmaceutical compositions which contain them in association with a compatible carrier.

Pharmaceutical preparations for systemic administration can be prepared, for example, by adding a compound of this invention as the active ingredient to pharmaceutically acceptable, non-toxic, inert, solid or liquid carriers

which are usual in such preparations. The pharmaceutical preparations can be administered enterally, parenterally or topically. Suitable preparations for enteral administration are, for example, tablets, capsules, dragees, syrups, suspensions, solutions and suppositories. Suitable pharmaceutical preparations for parenteral administration are infusion solutions.

The pharmaceutical preparations can contain, in addition to the active compounds of this invention, pharmaceutically acceptable inert or pharmacodynamically active additives. Tablets or granules, for example, can contain pharmaceutically acceptable binders, fillers, carrier materials or diluents. Liquid preparations, for example, can take the form of sterile water-miscible solutions. Capsules can contain a pharmaceutically acceptable filler or thickener. Furthermore, pharmaceutically acceptable flavor-improving additives and pharmaceutically acceptable substances commonly used as preservatives, stabilisers, moisture retainers or emulsifiers, salts for varying the osmotic pressure, buffers and other pharmaceutically acceptable additives can also be present in the pharmaceutical preparations.

The aforementioned pharmaceutically acceptable carrier materials and diluents are well known in the pharmaceutical compounding art; they can be organic or inorganic substances such as water, gelatin, lactose, magnesium stearate, talc, gum arabic, polyalkyleneglycols and the like.

For topical administration, the compounds of this invention are expediently made up in the form of ointments, salves, gels, tinctures, creams, solutions, lotions, sprays, suspensions, transdermal devices and the like. Ointments, creams and lotions are preferred. These pharmaceutical preparations for topical administration can be

prepared by mixing a compound of this invention as the active ingredient with pharmaceutically acceptable non--toxic, inert, solid or liquid carriers which are customary in such preparations and which are suitable for topical administration.

A conventional pharmaceutically acceptable antioxidant, e.g. tocopherol, N-methyl-γ-tocopheramine, butylated hydroxyanisole or butylated hydroxytoluene can also be incorporated into the pharmaceutical preparations containing the compounds of this invention.

So the compounds of this invention may be administered enterally, parenterally or topically. The dosages will vary according to the judgement of the attending clinician taking into account the mode of administration, the condition being treated and the requirements of the patient. Oral administration may require from about 5 mg. to about 200 mg. of the compounds daily in one or more dosages. A preferred oral dosage form is capsules containing from about 10 mg. to about 100 mg. of active ingredient. For topical administration, preferred dosage forms are solutions containing the active ingredient, e.g. at from 0.01% by weight to about 0.3% by weight, preferably from about 0.02% by weight to about 0.1% by weight, and lotions, gels, ointments and creams containing from about 0.5% by weight to about 5% by weight, preferably from about 0.1% by weight to about 2.0% by weight of active ingredient.

The compounds of formula I and their salts with cations (when R" is a hydroxy group) can be prepared by standard methods, in particular by a reaction in which a carbon-carbon double bond is formed, such as a Wittig reaction.

The invention therefore provides a process for the preparation of a compound of the formula I defined

above, or of a pharmaceutically acceptable salt thereof when R" is a hydroxy group,

which comprises reacting together compounds of the following formulae II and III:

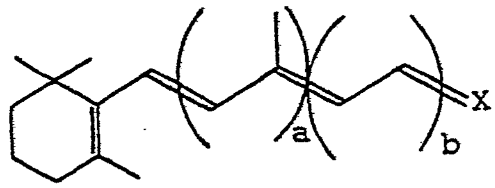

II

and

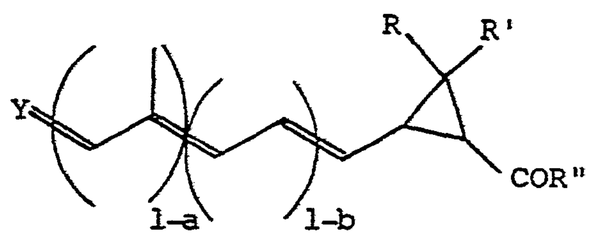

III

wherein R and R' are as defined above and R" is lower alkoxy,

a and b are each 0 or 1, with the proviso that, when a is 0, b is not 1;

one of X and Y is an oxygen atom and the other is either a phosphonato group that is singly bonded to the adjacent carbon atom, the other bond of the double bond pair at that carbon atom being a unit negative charge that is compensated by the presence of one unit of a cation, or a phosphonio group that is doubly bonded to the adjacent carbon atom,

in the presence of an inert organic solvent;

whereafter the resulting lower alkanoate is if desired converted into the free acid or an N-R'''-amide.

Preferably a and b are both 1; i.e., the compounds of the formulae II and III are respectively

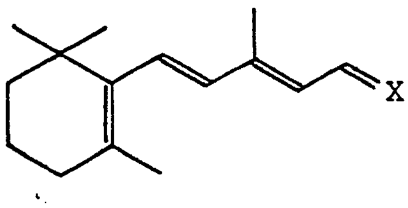

IIA

and

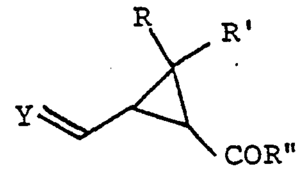

IIIA

so that the carbon-carbon double bonds in the compound of the formula IIA both have the desired trans-configuration. In the compounds of formulae II, IIA, III and IIIA, Y is preferably an oxygen atom and X is preferably a group

$$Z^1Z^2Z^3P$$

wherein each of $Z^1$, $Z^2$ and $Z^3$ is an organic hydrocarbon group, especially a lower alkyl or aryl group, e.g. phenyl, or two of them are lower alkoxy groups and one is a monovalent oxygen ion ($-O^-$), in association with the aforesaid unit of a cation, e.g. $Na^+$.

A particularly preferred embodiment of this process comprises reacting a phosphonium salt of the formula

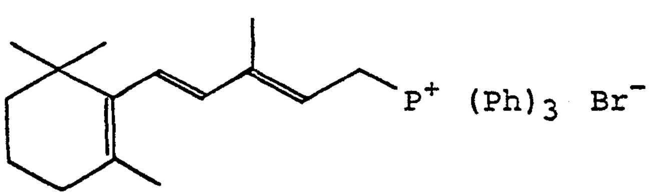

IIB

with a base to form the corresponding phosphorane, then reacting the phosphorane in situ with a cyclopropane aldehyde of the formula

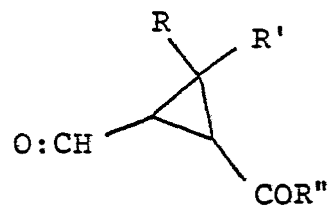

III B

wherein R, R' and R" are as hereinabove defined for this process, to yield a cyclopropyl-substituted polyene of the formula

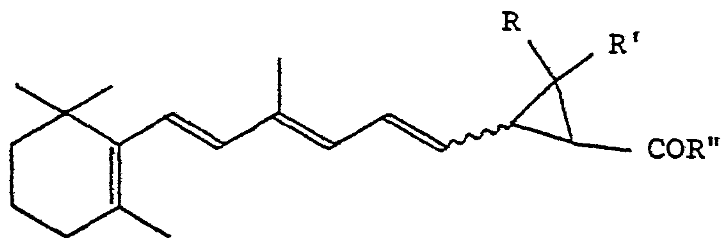

IA;

two preferred methods for carrying out this process are:

Method A

The reaction is conducted in the cold, about -20°C. to about 0°C., in an appropriate organic solvent such as dichloromethane in the presence of an alkali metal salt of a lower alkoxide. The reaction is completed in about one hour. Because of the solvent used, the product is predominantly the 2,3-trans-isomer of the formula

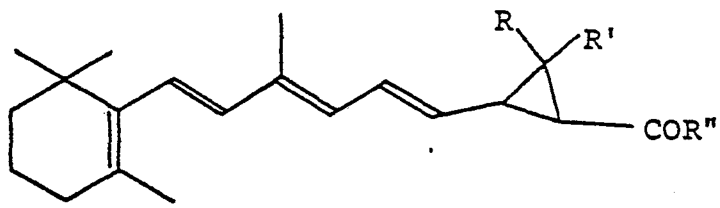

IB.

Method B

A suspension of the phosphonium salt in an appropriate non-isomerising solvent such as ether is treated with a hexane solution of an organo-lithium reagent such as *n*--butyllithium at -30°C. The cyclopropyl-aldehyde reactant is added and the solution is allowed to warm to 0°C. over one hour. The phosphonium salt and cyclopropyl aldehyde are known compounds or can be made from known compounds by methods known in the art.

2,3-*cis*- cyclopropane compounds of the formula I, i.e., having the formula

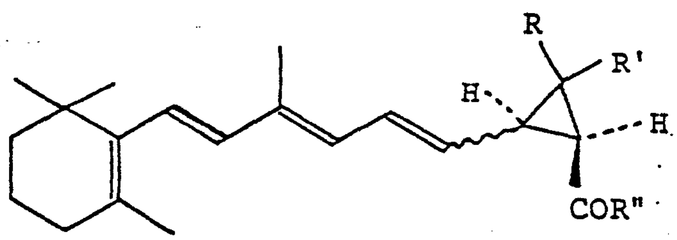

IC

wherein R and R' are as defined above and R" is a lower alkoxy group, and the wavy line between the cyclopropane ring and the terminal double bond indicates that that double bond may have the E- or Z -configuration,

can be prepared by Method B from a *cis*-cyclopropane-aldehyde ester.

The starting materials for this process, e.g. the phosphonium salt of the formula IIB and the cyclopropane--aldehyde ester of the formula IIIB, are known compounds or can be readily prepared from known compounds by standard methods.

The products resulting from either Method A or Method B can be purified e.g. by chromatography; the pure esters can if desired be hydrolysed to the corresponding

acids or be converted into the amides (where R" is NHR"') by methods known in the art. The esters can be converted directly into the amides by reaction with an amine $R'''NH_2$ or indirectly through the acids: thus the acids can be converted into a mixed anhydride with isobutylchloroformate in the presence of a tertiary amine, and the mixed anhydride can be reacted with an amine $R'''NH_2$ to form the amide; or the acids can be esterified to an activated ester (e.g. succinoyloxy ester) which can be reacted with the amine $R'''NH_2$, or coupled with that amine in the presence of dicyclohexylcarbodiimide. The acids can in turn be converted into their pharmaceutically acceptable salts by conventional methods.

The compounds of this invention can occur as isomers at the newly formed olefinic linkage, in particular at the 4,5-double bond when the starting materials have the formulae IIA and IIIA, and can be separated into the 4E and 4Z compounds or isomerised to the all E compound by conventional methods.

The following Examples illustrate the preparation of compounds and compositions of this invention:

## Example 1

4-E and 4-Z-[2,3-trans-isopropylidene-7-methyl-9-(2,6,6-trimethyl-cyclohexene-1-yl)]nona-4,6,8-trienoic acid

A solution of 15 g. of [7-(2,2,6-trimethyl-cyclohexene--1-yl)-5-methyl-hepta-2,4,6-trienyl]-triphenylphosphonium bromide and 3.9 g. of ethyl trans-2,2-dimethyl-3-formyl-cyclopropane-carboxylate in dichloromethane (150 ml.) was cooled to -20°C. A solution of sodium methoxide (1.9 g.) in methanol (30 ml.) was added over 15 minutes. The reddish-orange solution was stirred at 0°C. for 1 hour, diluted with hexane-ethyl acetate (7:3, 300 ml.) and washed with water and dried. Removal of the solvent gave an orange solid which was extracted with ether. The

ether extracts were evaporated to give orange solids which were subjected to high pressure liquid chromatography in 1.5% ethylacetate-hexane.

Two fractions were obtained which were separately subjected to hydrolysis of the ester to give the corresponding carboxylic acid.

Thus, 1.76 g. of the fraction first eluted was treated with 54 ml. of a 1:1 mixture of DMSO-50% KOH under argon at room temperature for 4 hours. The reddish mixture was poured into a cold 1N HCl solution (60 ml.) and acidified with 5N HCl to pH 1. The resulting mixture was extracted with ether and the ether extracts were washed with water and then with brine, and dried. Evaporation of the solvent gave the 4-Z-isomer as a foam. Data were obtained on the ethyl ester:
$^{1}$H NMR ($CDCl_3$) $\delta$ 1.0 (6H, s, 2C-$CH_3$); 1.16 (3H, s, C-$CH_3$); 1.25 (3H, t, J = 7Hz, $CH_2$-$C\underline{H}_3$); 1.26 (3H, s, C-$CH_3$; 1.68 (3H, s, =C-$CH_3$); 1.90 (3H, s, =C-$CH_3$); 2.14 (1H, dd, J = 8.5, 5.0 Hz, H-3); 4.15 (2H, q, J = 7Hz, $C\underline{H}_2CH_3$); 5.46 (1H, dd, J = 8.5, 15.0 Hz, H-4); 5.8-6.3 (3H, m, H-6, H-8 and H-9); 7.60 (1H, dd, J = 15.0, 11.0 Hz, H-5); UV (MeOH) $\lambda$max 302 nm ($\varepsilon$ = 1.65 x $10^4$).

Similar treatment of the second fraction to elute (0.9 g.) gave the 4-E-acid as a foam. Data were obtained on the ethyl ester: $^{1}$H NMR ($CDCl_3$)
$\delta$ 1.00 (6H, s, 2C-$CH_3$); 1.16 (3H, s,=C-$C\underline{H}_3$); 1.24 (3H, t, J = 7Hz, $CH_2C\underline{H}_3$); 1.25 (3H, s,=C-$CH_3$); 1.66 (3H, s, =C-$CH_3$); 1.9 (3H, s, =C-$CH_3$); 2.39 (1H, dd, J = 9.0, 5.0 Hz, H-3); 4.15 (2H, q, J = 7Hz, $C\underline{H}_2CH_3$); 5.20 (1H, t, J - 9.0, 9.0Hz, H-4); 6.2-6.5 (4H, m, H-5, H-6, H-8 and H-9. UV (MeOH) $\lambda$max 300 nm($\varepsilon$= 1.94 x $10^4$).

In the following Examples of pharmaceutical compositions, "active ingredient" means a preferred compound of

this invention or an equivalent amount of any compounds within the scope of this invention:

Example 2

Cream

| | mg./g. |
|---|---|
| Active ingredient | 10.0 |
| Cetyl alcohol | 40.0 |
| Stearyl alcohol | 40.0 |
| Isopropylmyristate | 100.0 |
| Polyoxyethylene (2) monostearyl ether (Brij 72) | 10.0 |
| Polyoxyethylene (20) monostearyl ether (Brij 78) | 25.0 |
| Propylene glycol | 100.0 |
| Benzyl alcohol | 10.0 |
| Purified water q.s. ad | 1.0 g. |

Method of Manufacture

Melt together and heat to about 70°C. the cetyl alcohol, stearyl alcohol, Brij 72, Brij 78 and isopropyl myristate. Add the propylene glycol to water in a separate container, heat to 70°C., and dissolve in this aqueous phase the benzyl alcohol. Dissolve or suspend the active ingredient in the aqueous phase while stirring. Add the aqueous phase to the oil phase with agitation. Continue stirring while cooling the cream to room temperature.

Example 3

Gel

| | mg./g. |
|---|---|
| Active ingredient | 10.0 |
| Propylene glycol | 50.0 |
| Hydroxypropylcellulose | 25.0 |
| Ethyl alcohol q.s. ad | 1.0 g. |

Method of Manufacture

Disperse or dissolve the active ingredient in alcohol with agitation. Add the propylene glycol and then the hydroxypropylcellulose, maintaining agitation until the hydroxypropylcellulose is evenly dispersed. Cool the resulting gel to allow for completion of hydration.

Example 4

Lotion

| | mg./g. |
|---|---|
| Active ingredient | 10.0 |
| Ethyl alcohol | 450.0 |
| Polyethylene glycol 400 | 350.0 |
| Hydroxypropylcellulose | 5.0 |
| Propylene glycol q.s. ad | 1.0 g. |

Method of Manufacture

Dissolve or disperse the active ingredient in the solvent mixture of ethyl alcohol, polyethylene glycol 400 and propylene glycol with agitation. Then add hydroxypropylcellulose maintaining agitation, until the hydroxypropylcellulose is evenly dispersed.

Example 5

Ointment

| | mg./g. |
|---|---|
| Active ingredient | 10.0 |
| Mineral Oil | 50.0 |
| White Petrolatum q.s. ad | 1.0 g. |

Method of Manufacture

Melt the petrolatum with heat and stirring. Slurry the active ingredient in mineral oil and add to the melted petrolatum. Continue stirring, while cooling the ointment to room temperature.

CLAIMS

1. Compounds of the formula

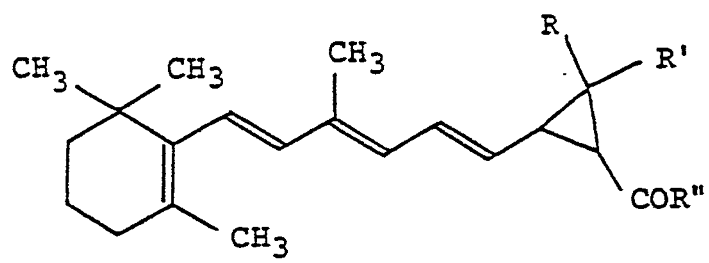

I

wherein

R and R' are independently hydrogen atoms or lower alkyl groups;

R" is a hydroxy or lower alkoxy group or a group -NHR''' wherein R''' is a hydrogen atom or a lower alkyl group;

and the pharmaceutically acceptable salts, formed with cations, of those compounds of formula I wherein R" is a hydroxy group;

wherein "lower alkyl" and "lower alkoxy" mean straight and branched chain alkyl and alkoxy groups that contain from 1 to 6 carbon atoms.

2. A compound of claim 1 in the form of the 4E isomer; especially 4-E-[2,3-trans-isopropylidene-7-methyl-9-(2,6,6-trimethyl-cyclohexen-1-yl)]nona-4,6,8-trienoic acid.

3. A compound of claim 1 in the form of the 4Z isomer; especially 4-Z-[2,3-trans-isopropylidene-7-methyl-9-(2,6,6-trimethyl-cyclohexen-1-yl)]nona-4,6,8-trienoic acid.

4. A compound of claim 1 in the form of the 2,3-cis--isomer.

5. A process for the preparation of compound as claimed in claim 1,

which comprises reacting together compounds of the following formulae II and III:

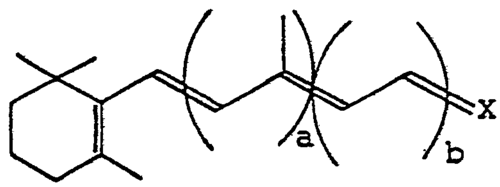

II

and

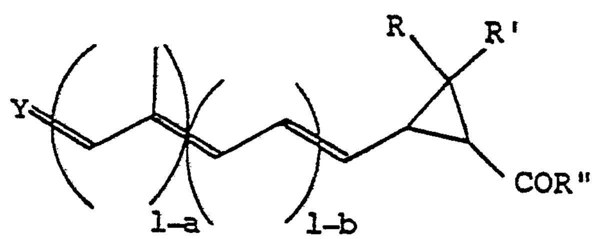

III

wherein R and R' are as defined in claim 1 and R" is lower alkoxy,

a and b are each 0 or 1, with the proviso that, when a is 0, b is not 1;

one of X and Y is an oxygen atom and the other is either a phosphonato group that is singly bonded to the adjacent carbon atom, the other bond of the double bond pair at that carbon atom being a unit negative charge that is compensated by the presence of one unit of a cation, or a phosphonio group that is doubly bonded to the adjacent carbon atom,

in the presence of an inert organic solvent;

whereafter the resulting lower alkanoate is if desired converted into the free acid or an N-R'''-amide.

6. A process as claimed in claim 5 wherein the compounds of the formulae II and III are respectively

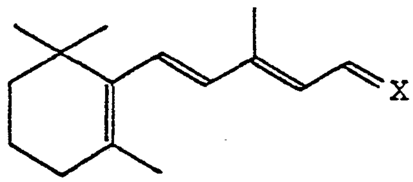

and

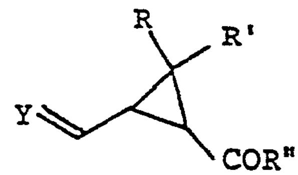

IIA IIIA,

the carbon-carbon double bonds in the compound of the formula IIA both having the desired <u>trans</u>-configuration;

in particular a process wherein, in the compounds of formulae IIA and IIIA, Y is an oxygen atom and X is a group

$$Z^1Z^2Z^3P$$

wherein each of $Z^1$, $Z^2$ and $Z^3$ is an organic hydrocarbon group or two of them are lower alkoxy groups and one is a monovalent oxygen ion ($-O^-$), in association with the aforesaid unit of a cation, more especially a process which comprises reacting a phosphonium salt of the formula

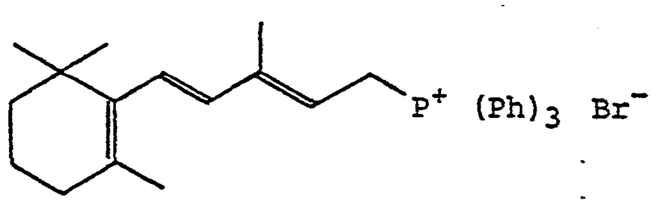 IIB

with a base to form the corresponding phosphorane, then reacting the phosphorane <u>in situ</u> with a cyclopropane aldehyde of the formula

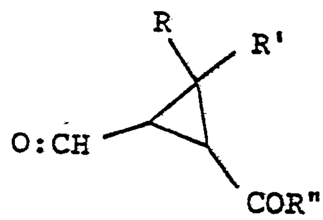

IIIB

wherein R, R' and R" are as defined above , to yield a cyclopropyl-substituted polyene of the formula

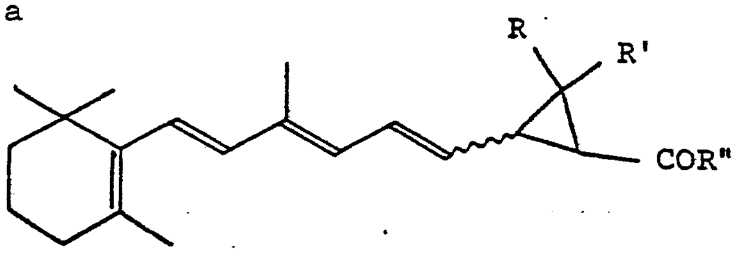

IA.

7. A pharmaceutical composition suitable for the treatment of dermatoses, which contains as active ingredient a compound as defined in claim 1, together with a compatible carrier.

8. Compositions as claimed in claim 7 in the form of dosage units, especially compositions containing 10 mg. to 100 mg. of active ingredient per dosage unit.

9. Compositions as claimed in claim 12 adapted for topical administration, in particular in the form of solutions preferably containing from 0.01% to 0.3% by weight, or in the form of lotions, gels, ointments or creams preferably containing 0.5% to 5% by weight, of active ingredient.

10. A composition as claimed in any of claims 7 to 9 wherein the active ingredient is 4-Z-[2,3-trans--isopropylidene-7-methyl-9-(2,6,6-trimethylcyclohexen--1-yl)]nona-4,6,8-trienoic acid or 4-E-[2,3-trans--isopropylidene-7-methyl-9-(2,6,6-trimethyl-cyclohexen--1-yl)]nona-4,6,8-trienoic acid.

11. A method of treating dermatoses comprising administering to a warm-blooded animal having a dermatosis an amount that is effective for treating dermatoses of a compound claimed in claim 1, together with a non-toxic pharmaceutically acceptable carrier.

European Patent Office

# PARTIAL EUROPEAN SEARCH REPORT

which under Rule 45 of the European Patent Convention shall be considered, for the purposes of subsequent proceedings, as the European search report

0113864

Application number

EP 83112400.3

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 3) |
|---|---|---|---|
| A | CH - A - 576 418 (SUMITOMO)<br>* Claim 1; columns 3,4 *<br>-- | 1,5 | C 07 C 175/00<br>A 61 K 31/19<br>A 61 K 31/16<br>A 61 K 31/215 |
| A | US - A - 4 206 140 (J. MARTEL et al.)<br>* Claims 1; columns 5-8 *<br>-- | 1,5 | |
| A | EP - A1 - 0 031 915 (BAYER)<br>* Claim 1; page 15 *<br>-- | 1,5 | |
| A | EP - A1 - 0 018 532 (BAYER)<br>* Claims 1,4,5 *<br>-- | 1,5 | |
| A | CH - A - 631 430 (BAYER)<br>* Claim 1 *<br>-- | 1 | |

TECHNICAL FIELDS SEARCHED (Int. Cl. 3)

C 07 C 175/00
C 07 C 61/00
C 07 C 69/00
C 07 C 103/00
C 07 C 177/00

## INCOMPLETE SEARCH

The Search Division considers that the present European patent application does not comply with the provisions of the European Patent Convention to such an extent that it is not possible to carry out a meaningful search into the state of the art on the basis of some of the claims.

Claims searched completely: 1-10

Claims searched incompletely: –

Claims not searched: 11

Reason for the limitation of the search: Method for treatment of the human or animal body by therapy (Article 52(4) of the European patent convention)

| Place of search | Date of completion of the search | Examiner |
|---|---|---|
| VIENNA | 16-03-1984 | PETROUSEK |

CATEGORY OF CITED DOCUMENTS

X : particularly relevant if taken alone
Y : particularly relevant if combined with another document of the same category
A : technological background
O : non-written disclosure
P : intermediate document
T : theory or principle underlying the invention
E : earlier patent document, but published on, or after the filing date
D : document cited in the application
L : document cited for other reasons
& : member of the same patent family, corresponding document

European Patent Office

**PARTIAL EUROPEAN SEARCH REPORT**

0113864

Application number

EP 83112400.3

## DOCUMENTS CONSIDERED TO BE RELEVANT

| Category | Citation of document with indication, where appropriate, of relevant passages | Relevant to claim | CLASSIFICATION OF THE APPLICATION (Int. Cl.³) |
|---|---|---|---|
| A | CH - A - 346 213 (BADISCHE ANILIN- & SODA-FABRIK)<br>* Page 1 *<br>-- | 1,5 | |
| A | DE - A1 - 2 431 930 (SANDOZ)<br>* Claim 1; page 4 *<br>---- | 1,5 | |
| | | | TECHNICAL FIELDS SEARCHED (Int Cl.³) |